(19)

Europäisches
Patentamt

European
Patent Office

Office européen
des brevets

(11) **EP 4 494 649 A1**

(12) **EUROPEAN PATENT APPLICATION**

(43) Date of publication:
**22.01.2025 Bulletin 2025/04**

(21) Application number: **23186402.6**

(22) Date of filing: **19.07.2023**

(51) International Patent Classification (IPC):
**A61K 38/16** (2006.01)     **C07K 14/47** (2006.01)
**C07K 14/16** (2006.01)     **A61P 25/00** (2006.01)
**A61P 25/02** (2006.01)

(52) Cooperative Patent Classification (CPC):
**A61K 38/16; C07K 14/47;** C12N 2750/14143

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Universität zu Köln**
**50931 Köln (DE)**

(72) Inventors:
• **Fischer, Dietmar.**
**41541 Dormagen (DE)**

• **Gobrecht, Philipp.**
**42105 Wuppertal (DE)**
• **Gebel, Jeannette.**
**40764 Langenfeld (Rheinland) (DE)**

(74) Representative: **Michalski Hüttermann & Partner
Patentanwälte mbB
Kaistraße 16A
40221 Düsseldorf (DE)**

Remarks:
The complete document including Reference
Table(s) and the Sequence Listing(s) can be
downloaded from the EPO website

(54) **PEPTIDES FOR USE IN THE TREATMENT OF AXONAL DAMAGE IN DIABETES**

(57)     The present invention relates to a peptide comprising the amino acid sequence LVESCKEAFWDRCLSVINLMSSKM according to SEQ ID No. 1 wherein the peptide is linked to a trans-activator of transcription (TAT) domain, or a pharmaceutically acceptable salt thereof, and its use in the treatment of axonal damage in diabetes.

**EP 4 494 649 A1**

**Description**

The present invention relates to peptides that are usable for the treatment of axonal damage in diabetes.

Diabetes mellitus is one of the most prevalent diseases in developed countries and is often associated with severe secondary conditions, such as diabetic peripheral neuropathy. Diabetes mellitus damages the nervous system and impairs axon regeneration, often leading to permanent functional loss. The mechanisms underlying the reduced regenerative capacity in diabetes are still poorly understood.

Zheng et al., Journal of Biological Chemistry, 285 (44): 34102-34212 (2010) describe a 24-residue peptide (p5), derived from p35, a Cyclin-dependent kinase 5 (Cdk5) neuronal activator, that specifically inhibits Cdk5-p25 hyperactivity and Tau hyperphosphorylation. Also, further peptides derived from CIP (Cdk5 Inhibitory Peptide), a 125-amino acid truncated peptide from p35 (residues 154-279), denoted p1, p2, p3 and p4 that also inhibit Cdk5-p25 activity, are described. It is suggested that stress-induced cleavage of the activator p35 to p25 and a p10 N-terminal domain induces the deregulation of Cdk5 hyperactivity. The 24-residue peptide, p5, spans the residues Lys(245)-Ala(277) of CIP. While p5 is described to more effectively inhibit Cdk5-p25 activity than CIP *in vitro,* it was found that in embryonal neuronal cells, p5 inhibited deregulated Cdk5-p25 activity but did not affect the activity of endogenous Cdk5-p35.

Zheng et al. also in PLoS ONE 8. 10.1371/journal.pone.0063332 (2013) describe that the Cdk5 Inhibitory Peptide (CIP) competitively binds to Cdk5 to lower its activity.

BK Binukumar et al., J Alzheimers Dis. 2014; 39(4): 899-909, describe a peptide denoted TFP5 to rescue cortical neurons *in vitro* and suggest that the peptide may be a novel candidate for type 2 diabetes therapy. TFP5 is described as a 24-amino acid peptide including a portion denoted P5 from the Cdk5 activator p35 (neuronal-specific p35 protein), modified with a Fluorescein-5-isothiocyanate (FITC) tag and an 11 amino acid transactivator of transcription (TAT) sequence, to penetrate and overcome the blood-brain barrier.

US 8,597,660 B2 describes that the peptide TFP5 can specifically inhibit a deregulated activity of Cdk5 responsible for neuronal pathology and prevent or reduce memory deficit associated with a neurodegenerative disease such as Alzheimer's disease.

Still, no therapeutic approaches are currently available to counteract the reduced axonal regenerative capacity required for nerve repair in diabetes.

Therefore, the object underlying the present invention was to provide compounds usable in axonal regeneration in diabetes.

The problem is solved by a peptide comprising the amino acid sequence LVESCKEAFWDRCLSVINLMSSKM according to SEQ ID No. 1 wherein the peptide is linked to a trans-activator of transcription (TAT) domain, or a pharmaceutically acceptable salt thereof.

Surprisingly, it was found that the peptide, after intrathecal injection, could promote axonal regeneration of motor, sensory and sympathetic fibers in the diabetic nerve. Thus, the peptide comprising the amino acid sequence LVESCKEAFWDRCLSVINLMSSKM according to SEQ ID No. 1 provides a promising pharmacological treatment for diabetic nerve damage. Advantageously, the peptide can provide a systemically pharmaceutically, intravenously applicable active peptide usable to promote axonal regeneration in diabetes.

Without being bound to a specific theory, it is assumed that the advantageous effects of the peptide are based on its ability to inhibit the interaction of Cdk5 and p35, which is unexpected, as the general opinion taught that Cdk5-p35 interaction was not affected by P5.

The amino acid sequence LVESCKEAFWDRCLSVINLMSSKM (SEQ ID No. 1) is linked to a TAT domain. The term "linked" as used herein refers to that the peptide may be directly linked or fused via amino acid binding or that the peptide alternatively may be linked to a TAT domain via a linker. The term "Linker" as used herein, refers to a relatively short series of amino acids, such as, between 2 and 10 amino acids that separate the amino acid sequence of SEQ ID No. 1 and the TAT domain. In embodiments, the amino acid sequence of SEQ ID No. 1 is directly fused to the TAT domain via amino acid binding.

TAT domains are derived from the human immunodeficiency virus's transactivator of transcription (TAT) and are cell-penetrating sequences. A minimal cell-penetrating sequence of TAT is the 9 amino acid sequence RKKRRQRRR (SEQ ID NO: 12), but longer fragments of TAT have also been demonstrated to mediate membrane translocation. TAT domains containing fusion are usable as therapeutic moieties. In embodiments, the TAT domain is selected from the group comprising YARAARRAARR (SEQ ID No. 2), YGRKKRRQRRR (SEQ ID No. 9), GRKKRRQRRR (SEQ ID NO: 10), GRKKRRQRRRPQ (SEQ ID NO: 11) and RKKRRQRRR (SEQ ID NO: 12). A preferred TAT domain is the sequence YARAARRAARR (SEQ ID No. 2). This domain effectively translocates neuronal cells. A TAT domain may preferably be located C-terminally.

In embodiments, the peptide comprises or consists of the amino acid sequence LVESCKEAFWDRCLSVINLMSSKMYARAARRAARR (SEQ ID No. 3). The peptide of SEQ ID No. 3 provided effective axonal regeneration of motor, sensory and sympathetic fibers in mice.

As used herein, the term "peptide" is understood to refer to synthetic or non-synthetic peptide compounds as well

**EP 4 494 649 A1**

as purified and modified fragments of natural proteins, native forms, or recombinant peptides.

Likewise, peptide is usable in form of pharmaceutically acceptable salts thereof. The term "pharmaceutically acceptable salts" refers to salts prepared from pharmaceutically acceptable non-toxic bases or acids. A pharmaceutically acceptable salt can be conveniently prepared from pharmaceutically acceptable non-toxic bases, including inorganic and organic bases. Preferred salts derived from inorganic bases include ammonium, calcium, magnesium, potassium, and sodium salts. Salts derived from pharmaceutically acceptable organic, non-toxic bases include salts of primary, secondary, and tertiary amines and cyclic amines. Preferably, the pharmaceutically acceptable salt is selected from the group of alkaline and earth-alkaline salts, particularly sodium or potassium salts. Also, calcium or magnesium salts can be preferred. Generally, peptides may be synthesized by standard solid-phase peptide synthesis, and reagents for peptide synthesis are commercially available.

A further aspect of the present invention relates to the peptide according to the invention for use as a medicament. For the description of the peptide, reference is made to the description above. Preferred peptides comprise a TAT domain. In preferred embodiments, a peptide LVESCKEAFWDRCLSVINLMSSKMYARAARRAARR, according to SEQ ID No. 3, may be used.

The peptide was found to enable and accelerate the anatomical regeneration of motor, sensory, and sympathetic fibers in the diabetic sciatic nerve after intrathecal and intravenous injection. This is assumed based on reduced CRMP2 inactivation, as shown in murine dorsal root ganglia (DRG) neurons. This enables a medicinal treatment with the peptide that can reduce one of the severe effects of diabetes. Thus, the peptide provides the advantage of a treatment option to overcome the regenerative deficiency in diabetes. In an embodiment, the peptide is for use in the treatment of axonal damage in diabetes.

A further aspect of the present invention relates to an expression vector expressing a nucleic acid encoding the peptide according to the invention, for use in the treatment of axonal damage in diabetes.

Known virus-based vectors for gene delivery and expression in mammalian cells, including vectors based on DNA viruses and RNA viruses, are usable. Usable vectors may be selected from herpes simplex virus (HSV) expression vectors, Epstein-Barr virus (EBV) expression vectors, Simian virus 40 (SV40) expression vectors, Adenovirus (Ad) expression vectors, Adeno-associated virus (AAV) expression vectors, Vaccinia virus (VV) expression vectors, or baculovirus. Preferred expression vectors are baculoviral or adeno-associated virus (AAV) expression vectors. Adeno-associated virus vectors are the leading platform for gene delivery for treating various human diseases and provide good safety profile.

In embodiments, the axonal damage in diabetes is diabetic peripheral neuropathy. Peripheral neuropathy is the most common type of neuropathy, referring to nerve damage outside the brain and spinal cord. Diabetic peripheral neuropathy particularly affects the nerves in feet and hands and can manifest as motor neuropathy, sensory neuropathy, or both. In preferred embodiments, the peptide or the vector described above is for use in promoting axonal regeneration in diabetic peripheral neuropathy.

According to the invention, the peptide can be formulated as a pharmaceutical composition. For use as a medicament the peptide can be used or included in a pharmaceutical composition. Accordingly, a further aspect relates to a pharmaceutical composition comprising as an active ingredient a peptide according to the invention or a pharmaceutically acceptable salt thereof. The pharmaceutical composition and any other pharmaceutical composition described herein can, optionally, comprise pharmaceutically acceptable carriers, excipients and/or vehicles. For the description of the peptide, reference is made to the description above. The pharmaceutical composition can be used to manufacture a medicament or as a medicament. In preferred embodiments, the pharmaceutical composition comprises the peptide LVESCKEAFWDRCLSVINLMSSKMYARAARRAARR according to SEQ ID No. 3.

In preferred embodiments, the pharmaceutical composition comprising as an active ingredient a peptide as described herein or a pharmaceutically acceptable salt thereof is for use in the treatment of axonal damage in diabetes.

The pharmaceutical composition preferably is a medicament for promoting axonal regeneration in diabetes.

The peptide can be solved or dispersed in a pharmaceutically acceptable carrier. The term "pharmaceutically or pharmacologically acceptable" refers to molecular entities and compositions that do not produce an adverse, allergic, or other untoward reaction when administered to a subject, such as, for example, a human, as appropriate. The pharmaceutical carrier can be, for example, a solid, liquid, or gas. Suitable carriers and adjuvants can be solid or liquid and correspond to the substances ordinarily employed in formulation technology for pharmaceutical formulations. For example, water, glycols, oils, alcohols, and the like may be used to form liquid preparations such as solutions. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen.

The pharmaceutical composition can be suitable for oral or parenteral administration. Parenteral administration includes subcutaneous, intramuscular, intravenous, intraneural, periradicular, intraperitoneal, and local administration. Specifically, the pharmaceutical composition may be administered systemically, for example, orally or intraperitoneally.

In embodiments, the pharmaceutical composition is formulated for topical or local application, such as intraneural or periradicular application, or for systemic application, such as intramuscular, intraperitoneal, intravenous, subcutaneous, intrathecal, or oral application. For treatment of peripheral nerves, the composition may be administered systemically or intraneurally into a nerve. Also, local or topical application may be preferred.

The pharmaceutical compositions may be conveniently presented in unit dosage form and prepared by any methods well-known in the art of pharmacy. The pharmaceutical composition may be produced under sterile conditions using standard pharmaceutical techniques well known to those skilled in the art.

In further embodiments, the pharmaceutical composition is formulated for intraocular application or a topic application, such as in the form of eye drops. Administration of eye drops may be applicable for diabetic patients and improve compliance. Compositions suitable for injectable use include sterile aqueous solutions or dispersions.

The present invention also relates to a use of the peptide as described herein for the manufacture of a medicament, particularly for use in the treatment of axonal damage in diabetes. For the description of the peptide, reference is made to the description above. In preferred embodiments, the peptide is the peptide according to SEQ ID No. 3. Further, for the description of a medicament and axonal damage in diabetes, reference is made to the description above.

A further aspect of the present invention relates to a method of treating axonal damage in diabetes, the method comprising the step of administering to a subject a therapeutically effective amount of a peptide as described herein. For medical purposes, subjects include human subjects and animal subjects, particularly mammalian subjects such as human subjects, mice, or rats. The term "therapeutically effective amount" is used herein to mean an amount or dose sufficient to cause an improvement in a clinically significant condition in the subject. For the description of the peptide, reference is made to the description above. In preferred embodiments, the peptide is the peptide according to SEQ ID No. 3. Further, for the description of axonal damage in diabetes, reference is made to the description above. In embodiments, the axonal damage is diabetic peripheral neuropathy.

The treatment may be a continuous prolonged treatment, or include a single or few-time administration. It was found that already one single injection was sufficient to significantly accelerate functional regeneration of the diabetic nerve *in vivo* compared to vehicle-treated control.

A further aspect relates to a peptide selected from the group comprising P5 (SEQ ID No. 4), P4 (SEQ ID No. 5), P3 (SEQ ID No. 6), P2 (SEQ ID No. 7) and P1 (SEQ ID No. 8), where the peptide is linked to a trans-activator of transcription (TAT) domain for use in the treatment of axonal damage in diabetes. Reference for the membrane-permeable TAT domain and possible linkers is made to the description above. In preferred embodiments, the peptide is selected from P5 (SEQ ID No. 4) and P3 (SEQ ID No. 6).

P5 has the following sequence: KEAFWDRCLSVINLMSSKMLQINA (SEQ ID No. 4). P4 has the following sequence: ELQAVLLTCLYLSYSYMGNEISYPLKPFL (SEQ ID No. 5). P3 has the following sequence: ELQAVLLTCLYLSYSYMGNEISYPLKPFLVESCKEAFWDRCLSVINL (SEQ ID No. 6). P2 has the following sequence: MGNEISYPLKPFLVESCKEAFWDRCLSVINLMSSKMLQINA (SEQ ID No. 7). P1 has the following sequence:
ELQA VLL TCL YLSYSYMGNEISYPLKPFL VESCKEAFWDRCLSVINLMSSKMLQINA (SEQ ID No. 8).

In an embodiment, the peptide P5 linked to a trans-activator of transcription (TAT) domain has the sequence KEAFWDRCLSVINLMSSKMLQINA Y ARAARRAARR (SEQ ID No. 13).

A related aspect refers to an expression vector expressing a nucleic acid encoding a peptide selected from the group comprising P5 (SEQ ID No. 4), P4 (SEQ ID No. 5), P3 (SEQ ID No. 6), P2 (SEQ ID No. 7) and P1 (SEQ ID No. 8), where the peptide is linked to a trans-activator of transcription (TAT) domain, for use in the treatment of axonal damage in diabetes. In embodiments, the axonal damage in diabetes is diabetic peripheral neuropathy.

The peptides can be formulated as a pharmaceutical composition. Accordingly, a further aspect relates to a pharmaceutical composition comprising as an active ingredient a peptide selected from the group comprising P5 (SEQ ID No. 4), P4 (SEQ ID No. 5), P3 (SEQ ID No. 6), P2 (SEQ ID No. 7) and P1 (SEQ ID No. 8), where the peptide is linked to a trans-activator of transcription (TAT) domain, for use in the treatment of axonal damage in diabetes. The pharmaceutical composition can, optionally, comprise pharmaceutically acceptable carriers, excipients, and/or vehicles. The pharmaceutical composition preferably is a medicament for promoting axonal regeneration in diabetes. For the pharmaceutical composition, its formulation, and application, reference is made to the description above. Preferably, the pharmaceutical composition is formulated for intraocular application, such as in the form of eye drops.

This aspect also relates to a use of the peptide selected from the group comprising P5 (SEQ ID No. 4), P4 (SEQ ID No. 5), P3 (SEQ ID No. 6), P2 (SEQ ID No. 7) and P1 (SEQ ID No. 8), where the peptide is linked to a trans-activator of transcription (TAT) domain, or an expression vector expressing a nucleic acid encoding a peptide selected from the group comprising P5 (SEQ ID No. 4), P4 (SEQ ID No. 5), P3 (SEQ ID No. 6), P2 (SEQ ID No. 7) and P1 (SEQ ID No. 8), where the peptide is linked to a trans-activator of transcription (TAT) domain, for the manufacture of a medicament for the treatment of axonal damage in diabetes.

This aspect further relates to a method of treating axonal damage in diabetes, the method comprising the step of administering to a subject a therapeutically effective amount of a peptide selected from the group comprising P5 (SEQ ID No. 4), P4 (SEQ ID No. 5), P3 (SEQ ID No. 6), P2 (SEQ ID No. 7) and P1 (SEQ ID No. 8), where the peptide is linked to a trans-activator of transcription (TAT) domain, or an expression vector expressing a nucleic acid encoding a peptide selected from the group comprising P5 (SEQ ID No. 4), P4 (SEQ ID No. 5), P3 (SEQ ID No. 6), P2 (SEQ ID No. 7) and P1 (SEQ ID No. 8), where the peptide is linked to a trans-activator of transcription (TAT) domain.

Unless otherwise defined, the technical and scientific terms used herein have the same meaning as commonly understood by one of ordinary skill in the art to which this invention belongs.

The examples which follow serve to illustrate the invention in more detail but do not constitute a limitation thereof.

The figures show:

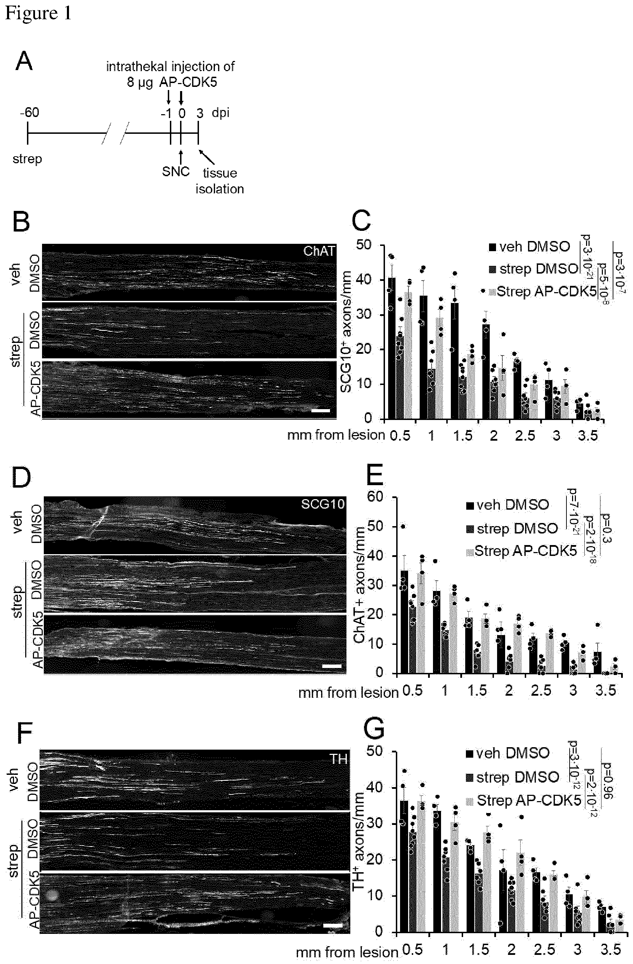

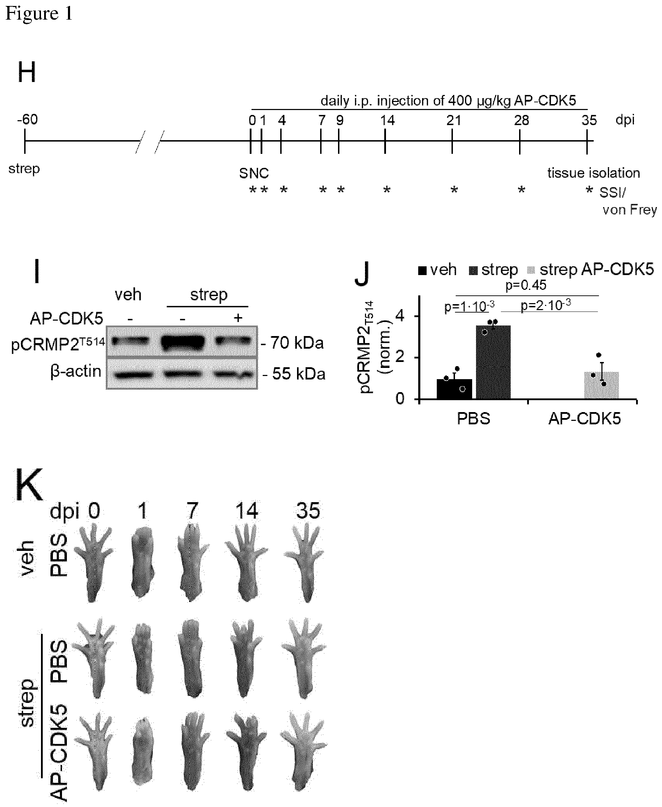

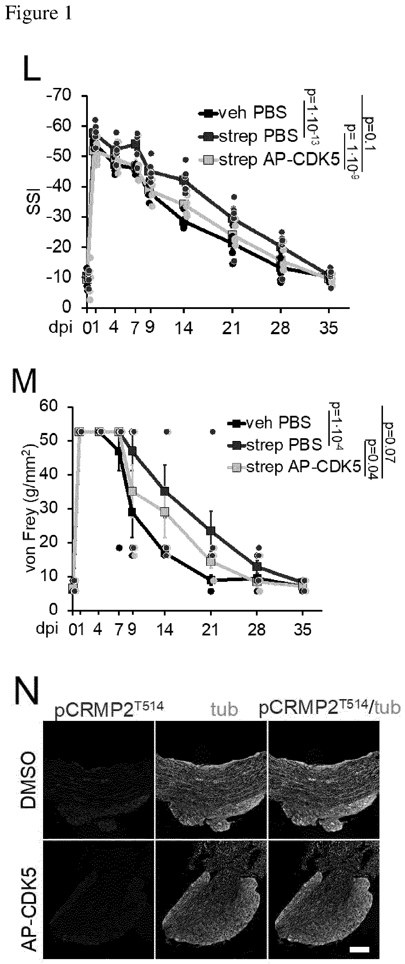

Figure 1    the effect of the peptide of SEQ ID No. 3 on diabetic axon regeneration. Figure 1A shows the timeline of experiments shown in B-G. Figure 1B shows representative images of axonal regeneration in the vehicle (veh) or strep-injected mice treated with DMSO or AP-CDKS 1 day before and on the sciatic nerve crush (SNC). Nerves were isolated 3 dpi and stained against choline acetyltransferase (ChAT) as a marker for motor fibers. Asterisks are marking the lesion site. Figure 1C shows the quantification of axon regeneration depicted in B. Figure 1D shows representative images of axon regeneration of sensory fibers stained against superior cervical ganglion 10 (SCG10) of the experimental conditions described in B. Asterisks mark the crush site. Figure 1E shows the quantification of axon regeneration depicted in D. Figure 1F shows representative images of axon regeneration of sympathetic fibers stained with tyrosine hydroxylase (TH) of the experimental conditions described in B. Asterisks mark the crush site. Figure 1G shows the quantification of axon regeneration depicted in F. Figure 1H shows the timeline of experiments shown in I-M. After SNC, SSI and von Frey tests were assessed at indicated time points (asterisks). Figure 1I shows a Western blot against $pCRMP2^{T514}$ of L4-L6 DRG from mice treated as described in H. $\beta$-actin served as a loading control. Figure 1J shows the quantification of Western blots depicted in I. Data were normalized against actin level and veh/PBS. Figure 1K shows representative pictures of the injured paw before and at 1, 7,14, 21, 28, and 35 days post-injury (dpi). Figure 1L shows functional motor recovery assessed by static sciatic index (SSI) at various time points. Figure 1M shows functional sensory recovery determined by the von Frey test. Figure 1N shows representative images of inhibitory phosphorylated CRMP2 ($pCRMP2^{T514}$, red) of DRG (L6) from strep-treated mice after i.p. DMSO or AP-CDKS injection. The tissue was harvested after completing functional recovery. $\beta$III-tubulin (tub; green) serves as a neuronal marker. The data represent mean$\pm$SEM (bars) and single values (dots) of B-G) four to eight (veh DMSO n=4; strep DMSO n=8; strep AP-CDK5 n=4), K) three independent samples (n=3) and L, M) six (n=6) animals. The significance of intergroup differences was evaluated by two-way (C, E, G) or three-way (L, M) analysis (ANOVA) with the Holm Sidak post hoc test. P-value indicates statistical significance. Scale bar: 100 $\mu$m.

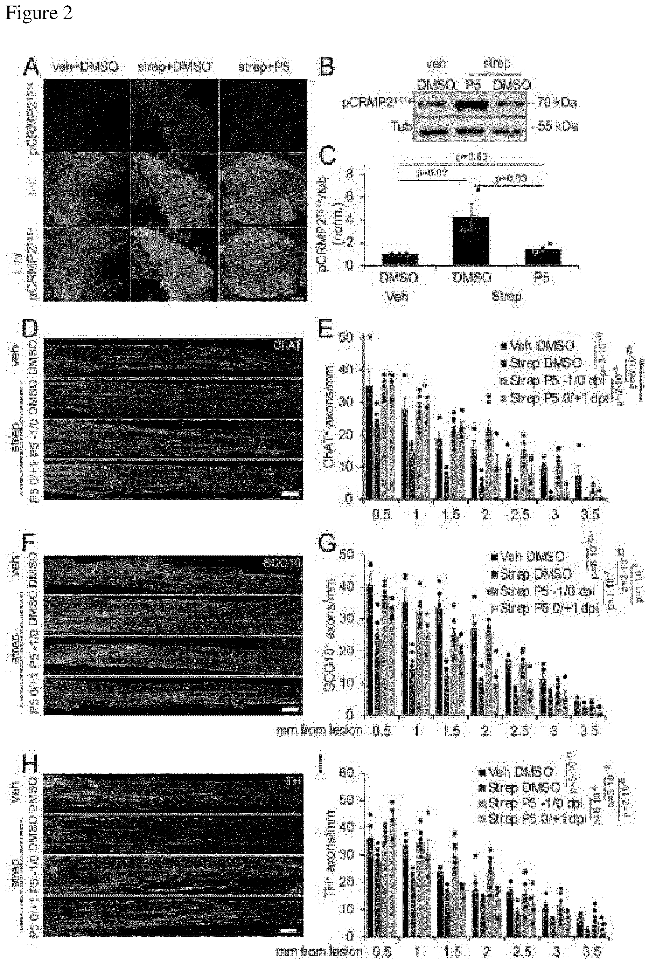

Figure 2    the effect of the peptide of SEQ ID No. 13 (denoted P5) on diabetic axon regeneration. Figure 2A shows representative images of the inhibitory phosphorylated CRMP2 ($pCRMP2^{T514}$) of DRG (L6) from strep-treated mice that developed diabetes after i.p. DMSO (vehicle) or peptide injection 1 day before and on the SNC. The tissue was harvested 3 days after the SNC. $\beta$III-tubulin (Tub) served as a neuronal marker. Figure 2B shows a Western blot against $pCRMP2^{T514}$ of L4-L6 DRG from mice treated as described. Figure 2C shows the quantification. Figure 2D shows representative images of axonal regeneration in the vehicle or strep-injected mice treated with DMSO or the peptide of SEQ ID No. 13 (denoted P5) 1 day before and on the day of the sciatic nerve crush (SNC). Nerves were isolated 3 dpi and stained against choline acetyltransferase (ChAT) as a marker for motor fibers. Asterisks mark the crush site. Figure 2E shows the quantification of axon regeneration depicted in Figure 2D. Figure 2F shows representative images of axon regeneration of sensory fibers stained against superior cervical ganglion 10 (SCG10). Asterisks marking the crush site. Figure 2G shows the quantification of axon regeneration depicted in Figure 2F. Figure 2H shows representative images of axon regeneration of sympathetic fibers stained with tyrosine hydroxylase (TH). Asterisks marking the crush site. Figure 2I shows the quantification of axon regeneration depicted in Figure 2H.

Examples

Materials and Methods:

Animals:

To model type 1 diabetes mellitus (T1DM), male and female wild-type Ola received a single intraperitoneal injection of 100 mg/kg streptozotocin (strep) or vehicle (veh; 0.1 M citrate buffer, pH 4.5) at 4-6 weeks of age. The efficacy of the treatment was confirmed by assessing blood glucose levels 1 week after injection. Strep mice with over 250 mg/dl of blood glucose were considered diabetic. Thus, diabetes was induced 2 months before sciatic nerve crush.

As a type 2 diabetes mellius (T2DM) model, db/db mice with a deficient leptin receptor of C57BL/6 genetic background were used as described by Coleman and Hummel, Diabetologia volume 3, pages 238-248 (1967). Heterozygous wt/db mice were used as controls since those mice showed no signs of diabetes. Mice were maintained in cages with one to five animals on a 12 h light/dark cycle *ad libitum* access to food and water.

Surgical procedures:

All surgical procedures on mice adhered to respective animal care guidelines and were approved by the local authorities (LANUV Recklinghausen). For intrathecal AAV2.1 (CRMP2T514A-HA, GFP, tdTomato, and Cre-GFP: titer: ≥7·1012 vg/ml, Addgene), a 5 mm skin incision was made above the L4-L5 spines. The muscle tissue was carefully spread, and 2.5 μl of the viral solution was injected into the cerebrospinal fluid between the L4 and L5 spines using a Nanoject II injector (Drummond Scientific)
with three consecutive 833 nl injections with a speed of 23 nl/s and 20 s intervals between injections. The skin was closed using 6-0 sutures. Transduction was performed 2 weeks before sciatic nerve crush (SNC). SNC was performed as described in Gobrecht et al., Nature Communications 5, 4561 (2014) and Gobrecht et al., (2016). In brief, animals were anesthetized by inhalation anaesthesia with 1.5% isoflurane in oxygen, and a skin incision of 5 mm was made above the gluteal region. The ischiocrural musculature was carefully spread, inducing minimal tissue damage to expose the right sciatic nerve from the sciatic notch to the point of trifurcation. Sciatic nerve crush injury was performed proximal to the tibial and peroneal divisions using Dumont #5 forceps (Hermle) for 20 s. The skin was then closed using 6-0 sutures.

Application of the peptide according to SEQ ID No. 3 (AP-CDK5):

The intrathecal application of anti-peptide against CDK5 (AP-CDK5) and P5 was performed with the above-described method to assess anatomical regeneration. Either 2 μl AP-CDK5 or P5 (each 4 mg/ml) or vehicle (DMSO) was injected the day before SNC and the day of SNC or the day of SNC and the day after. For analyses of functional recovery, AP-CDKS was injected daily at 400 μg/kg from the day of SNC until the mice were sacrificed. AP-CDKS and P5 (Genescript) were fused to a TAT-sequence for cell membrane penetration and had the following sequence: AP-CDKS: LVESCKEAFWDRCLSVINLMSSKMYARAARRAARR and (SEQ ID No. 3) P5: KEAFWDRCLSVINLMSSKMLQINAYARAARRAARR (SEQ ID No. 13).

Cloning and baculovirus production:

Genes were cloned from murine cDNA (brain or DRG) using the following primer: p35 (5' TAAGCCTTTACCCCCCTCCA 3' (SEQ ID No. 14), 5' ATCCTTGAGCCAGGATACCG 3' (SEQ ID No. 15)), CRMP2 (5' AGCTGTCCTCTTGAGATTACT 3' (SEQ ID No. 16), 5' GGAAGTCTCAGAATGTCCTC 3' (SEQ ID No. 17)). Baculoviruses were produced as described in Levin, E. et al., (2016), Sci Rep 6, 1-10.

Dorsal root ganglion (DRG) neuron cultures:

Since cultivated sensory neurons from T1DM mice do not display a diabetic phenotype in culture, only T2DM cells were used for cell culture experiments. Sensory neurons derived from dorsal root ganglia (DRG) were harvested from adult wt/db and db/db mice, as described in Gobrecht et al., Nature Communications 5, 1-10 (2014) and Gobrecht et al., 2016. Briefly, isolated DRG were incubated in DMEM containing 0.25% trypsin/EDTA (GE Healthcare) and 0.3% collagenase Type IA (Sigma Aldrich) for 45 min at 37°C, followed by mechanical dissociation. Cells were resuspended in DMEM containing 2% B27 (Thermo Fisher Scientific) and 500 U/ml penicillin/streptomycin (Merck, Millipore). Cells were cultivated at 37 °C and 5% $CO_2$ on poly-D-lysine (0.1 mg/ml; molecular weight <300,000 Da) and laminin (20 μg/ml; Sigma)- coated 8 well tissue culture chambers (Sarstedt) or 96 well plates (Nunc). Cells were transduced 5 h after plating with either GFP-, or CRMP2T/A-HA-expressing baculoviruses (BV). BV were produced as described in Levin, E. et al., (2016), Sci Rep 6, 1-10. The culture medium was changed at 16 h after transduction.

Immunocytochemical staining:

For assessing axon growth, fixed cells were stained for βIII-tubulin (1:1000; BioLegend, RRID:AB_2313773) was performed. Imaging and quantifying total axon length and neurons per well were automatically performed with the VS120 (Olympus) and the NeuriteTracer Imaged PlugIn. The average axon length per neuron and neuron counts per experimental group were normalized to the control group.
The number of pCRMP2-positive cells was determined after 48 h cultivation using antibodies against βIII-tubulin (1:1000; BioLegend, RRID:AB_2313773), pCRMP2T514 (1:500; Abcam, RRID:AB_942229) and pCRMP2S522 (1:500; Abcam, RRID:AB_2732884). The number of p35-positive cells was determined using antibodies against p35 (1:500; cell signaling, mAb 2680) and βIII-tubulin (1:1000; BioLegend, RRID:AB_2313773). The formula calculated the intensity:

$$\text{Intensity} = \text{integrated density of selected cell} - (\text{area of selected cell} \times \text{mean fluorescence of background}).$$

Cells were counted as positive when the intensity surpassed a chosen threshold.

The number of pMAP1B-positive axons tips containing detyrosinated tubulin was determined using antibodies against pMAP1B$^{T1265}$ (1:500; Thermo, RRID:AB_11152533) or detyrosinated tubulin (1:500; Millipore, RRID:AB_177350), respectively. Axon tips were defined as the last 15 μm of βIII-tubulin-positive neurite extensions. Axon tips deemed positive by the formula: Intensity=integrated density of axon tip - (length of axon tip x mean fluorescence of background) surpassed a chosen threshold.

Data presents the mean± SEM of at least three independent experiments (bars) and the mean of three wells with at least 25 neurons/axon tips per well of individual experiments (dots). Significances were evaluated using two-way ANOVA followed by the Holm Sidak post hoc test.

Western blot assays:

DRG (L3-L5) or 3 mm of the sciatic nerves proximal to the injury site from three mice per group were homogenized by sonication in 100 μl lysis buffer (20 mM Tris-HCl pH 7.5, 10 mM KCl, 250 mM sucrose, 10 mM NaF, 1mM DTT, 0.1 mM Na3VO4, 1% Triton X-100, 0.1% SDS, 1% protease inhibitors (Merck Millipore) and phosphatase inhibitors (Roche)). Lysates were centrifuged at 8,000 g for 10 min at 4 °C, and soluble proteins from the supernatant were separated by SDS-PAGE (precast protein gels, BioRad) followed by a transfer to nitrocellulose membranes (Bio-Rad) according to standard protocols. Blots were blocked in 5% dried milk in TBS-T and incubated with 5% BSA-containing antibodies against βIII-tubulin (1:5,000; BioLegend, RRID:AB_2313773), p35 (1:1,000; cell signaling, RRID:AB_1078214), pCRMP2$^{S522}$ (1:1,000; Abcam, RRID:AB_2732884), pCRMP2$^{T514}$ (1:1,000; Abcam, RRID:AB_942229), CRMP2 total (1:1,000, Abcam, RRID:AB_731750), or CDK5 (1:1000, Abcam, RRID:AB_726779) at 4°C overnight. All antibodies were detected with anti-rabbit or anti-mouse IgG secondary antibodies conjugated to HRP (1:80,000; Sigma). Antigen-antibody complexes were visualized using an enhanced chemiluminescence substrate (Bio-Rad) on the Fluorochem E imaging system (Protein Simple). All western blots were repeated at-least three times with independent samples.

Immunohistochemical staining:

DRG (L3-L5) and sciatic nerves were fixed in 4% PFA overnight at 4°C, followed by dehydration in 30% sucrose overnight at 4°C. Cryosections (14 μm for DRG, 20 μm for sciatic nerve) were permeabilized for 10 min in methanol, blocked (2% BSA, 5% donkey serum) for 1 h, then stained with antibodies either against βIII-tubulin (1: 1,000; BioLegend, AB_2313773), p35 (1:1,000; cell signaling, mAB_2680), pCRMP2$^{S522}$ (1:1,000; Abcam, AB_193226), pCRMP2$^{T514}$ (1:1,000; Abcam, AB_62478), CRMP2 total (1:1,000, Abcam, AB_36201), GFP (1:500, Novus, AB_10128178), HA (1:500, Sigma-Aldrich, AB_2600700), or CDK5 (1:1,000, Abcam, AB_40773). Antibodies were detected with anti-rabbit, anti-goat, or anti-mouse secondary antibodies conjugated to Dylight-405,_Alexa-594, or Alexa-488.

Representative pictures of each experimental group were taken on an SP8 confocal microscope (Leica, Wetzlar) or a VS120 (Olympus).

For assessment of anatomical regeneration, the sciatic nerves were isolated 3 days post-injury (dpi). The tissue was processed and stained as described above. For analyses of motor regeneration, the nerves were stained with choline acetyltransferases (ChAT; Millipore, 1:250; RRID:AB_2079751). Sensory regeneration was investigated with superior cervical ganglia 10 (SCG10; Novus; 1:500; RRID:AB_10011569). Regeneration of sympathetic fibers was quantified with immunostaining against tyrosine hydroxylase (TH; Novus, 1:500; RRID:AB_10077691).

For DAB stainings, sections were incubated in 0.0003% $H_2O_2$ for 10 min, followed by 1 h blocking (2% BSA, 5% Donkey Serum in PBS-T). Antibody incubation was performed at the above-mentioned concentration at 4 °C. Vectastain Elite ABC-HRP kit (Linaris) and DAB tablets (Merck) were used to visualize staining. Pictures were taken with a VS120 (Olympus).

For quantitative analysis of transduced neuromuscular junctions (NMJ), whole-mount staining of the *musculus hallucis longus* was performed. Muscles were isolated, fixed for 1 h in 4% PFA, and permeabilized in 2% Triton-x. The GFP antibody (1:500, Novus, ab10128178) was incubated overnight at 4 °C. The secondary antibody (anti-goat Alexa 488, 1: 1000; Thermo Fisher Scientific) and α-bungarotoxin-Alexa594 (BTX; 1: 1000, Invitrogen) were incubated for 1 h. Images were taken confocally with the SP8 (Leica).

To verify transduced axons in the skin, footpads innervated by the sciatic nerve were isolated 2 weeks after transduction. Cryosections (20 μm) were counterstained with DAPI, and pictures were taken with a VS120 (Olympus).

Static sciatic index (SSI):

Motor recovery after SNC was determined by calculating the SSI as described in Baptista AF et al., 2007, J Neurosci Methods 161, 259-264 and Gobrecht et al., Nature Communications 5, 1-10 (2014). At the same day time (1:00-2:00 -pm) at indicated time points after SNC, mice were lifted from the ground to photograph the hind paws. Toe spreading on contralateral (C, left) and ipsilateral (I, right) sides relative to the SNC was assessed by measuring paw_length (PL) and the_distance between the first and fifth toe (FF). The SSI was then calculated using the previously described formula: SSI = 101.3((IFF-CFF)/CFF)-54.03((IPL-CPL)/CPL)-9.5 in as described in Baptista AF et al., 2007, J Neurosci Methods 161, 259-264.

Data represent mean SSI±SEM per experimental group. Statistical significances of intergroup differences were evaluated using two-way ANOVA followed by the Holm Sidak *post hoc* test.

von Frey test:

Sensory recovery after SNC was determined at the indicated time points after SNC with the von Frey filament test as described in Gobrecht et al., 2014 and Gobrecht et al., (2016). Tests were performed at the same time of day by the same experimenter. Mice were placed on an elevated metal grid (pore size: 2 mm) and allowed to acclimate for 15 min before testing. Then, the smallest, differently sized, innocuous von Frey filaments (Muromachi Kikai) were consecutively poked into the ipsilateral hind paw to elicit a positive response, indicated by sharp paw withdrawal. Statistical significances of intergroup differences were evaluated using two-way ANOVA followed by the Holm Sidak *post hoc* test.

Example 1: Determination of the effect of the peptide of SEQ ID NO. 3 on anatomic regeneration in diabetic mice

1.1 Determination of the peptide of SEQ ID No. 3 on regeneration of motor, sensory, and sympathetic fibers in the diabetic sciatic nerve *in vivo*

The 35-amino-acid peptide of SEQ ID No. 3 directed against CDK5 was tested for its effect on axonal regeneration and phosphorylation of CRMP2 *in vivo.*

To verify the regeneration-stimulating effect of the peptide, axonal regeneration was determined *in vivo.* Figure 1A shows the timeline of experiments. Intra peritoneal strep injection induced T1DM two months before sciatic nerve crush (SNC). Intrathecal application of the peptide was performed as described above. Either 8 $\mu$g peptide or vehicle (DMSO) was injected 1 day prior and on the day of the SNC. Anatomical regeneration was assessed 3 days post-injury (dpi).

Figure 1B shows representative images of axonal regeneration in a vehicle or strep-injected mice treated with DMSO or the peptide of SEQ ID No. 3 (AP-CDK5) either 1 day before or on the day of the sciatic nerve crush (SNC). Nerves were isolated 3 dpi and stained against choline acetyltransferase (ChAT) as a marker for motor fibers. Asterisks mark the crush site. Figure 1C shows the quantification of axon regeneration depicted in Figure 1B.

Figure 1D shows representative images of axon regeneration of sensory fibers stained against superior cervical ganglion 10 (SCG10) of the experimental conditions as described. Asterisks mark the crush site. Figure 1E shows the quantification of axon regeneration depicted in Figure 1D.

Figure 1F shows representative images of axon regeneration of sympathetic fibers stained with tyrosine hydroxylase (TH) of the experimental conditions as described. Asterisks mark the crush site. Figure 1G shows the quantification of axon regeneration depicted in Figure 1F.

As can be taken from Figures 1 B-G, intrathecal injection of the peptide accelerated anatomical regeneration of motor, sensory, and sympathetic fibers in the diabetic sciatic nerve *in vivo.*

1.2 Determination of the effect of the peptide of SEQ ID No. 3 on the priming phosphorylation of collapsin response mediating protein 2 (CRMP2) *in vivo*

Figure 1H illustrates the timeline of experiments. Intraperitoneal (i.p.) strep injection induced T1DM two months before sciatic nerve crush (SNC). The peptide of SEQ ID No. 3 (denoted AP-CDK5) was injected i.p. every day from the SNC until the mice were sacrificed. After SNC, SSI and von Frey tests were assessed before the day of SNC, and days 1, 4, 7, 9, 14, 21, 28, and 35 thereafter. Figure 1I shows a Western blot against pCRMP2$^{T514}$ of L4-L6 DRG from mice treated as described. $\beta$-actin served as a loading control. Figure 1J shows the quantification of Western blots depicted in I. Data were normalized against actin levels and vehicle PBS.

As can be taken from Figures 1I and 1J, the peptide of SEQ ID No. 3 strongly reduced CRMP2 inactivation in DRG neurons. It is assumed that the peptide prevented Cdk5 activation, and as a result, CRMP2 was not primed, and consequently, the inactivation of CRMP2 was strongly reduced. As it is further assumed that CRMP2 phosphorylation compromises axonal growth by reducing microtubule polymerization, these results imply that the peptide can improve diabetic axonal regeneration.

1.3 Determination of the peptide of SEQ ID No. 3 on functional regeneration *in vivo*

In addition to the anatomical regeneration, as shown in Figures 1B to 1G, also functional regeneration was determined. Figure 1K shows representative pictures of the injured paw before and at 1, 7,14, 21, 28, and 35 days post-injury (dpi). As can be taken from Figure 1K the peptide of SEQ ID No. 3 (AP-CDK5) accelerated axon regeneration in diabetic mice. Figure 1L shows functional motor recovery assessed by static sciatic index (SSI) at various time points. Figure 1M shows functional sensory recovery determined by the von Frey test.

The Figures 1L and M show that the peptide can be applied systemically (intraperitoneally) and provide a functional effect. This is all the more important as therapy in this experiment only started post-injury.

Figure 1N shows representative images of inhibitory phosphorylated CRMP2 (pCRMP2$^{T514}$, red) of DRG (L6) from strep-treated mice after i.p. DMSO or peptide (AP-CDK5) injection.

The tissue was harvested after completing functional recovery. βIII-tubulin (tub) served as a neuronal marker.

As can be taken from Figure 1N, the peptide strongly reduced CRMP2 inactivation in neurons. It is assumed that the peptide prevented Cdk5 activation, and as a result, CRMP2 was not primed, and consequently, the inactivation of CRMP2 was strongly reduced. As it is further assumed that CRMP2 phosphorylation compromises axonal growth by reducing microtubule polymerization, also these results imply that the peptide can improve diabetic axonal regeneration.

In summary, these results show that the peptide of SEQ ID No. 3 can sustain CRMP2 activity in the diabetic peripheral nervous system (PNS) and thereby accelerate anatomical and functional regeneration. Thus, treatment with the peptide can promote axonal regeneration in diabetic peripheral neuropathy, and the peptide provides a promising therapeutical tool for treatment of axonal damage in diabetic peripheral neuropathy.

Example 2: Determination of the effect of the peptide of SEQ ID NO. 13 on anatomic regeneration in diabetic mice

The 35-amino-acid peptide of SEQ ID No. 13, P5 fused to a TAT sequence, directed against CDK5, was tested for its effect on axonal regeneration and phosphorylation of CRMP2 *in vivo.*

Intrathecal application of the peptide was performed as described above. Either peptide (4 mg/ml), streptozotocin (strep) or vehicle (DMSO) was injected the day before sciatic nerve crush (SNC) and the day of SNC or the day of SNC and the day after. For analyses of functional recovery, the peptide of SEQ ID No. 13 was injected daily at 400 μg/kg from the day of SNC until the mice were sacrificed.

The phosphorylation level of collapsin response mediating protein 2 (CRMP2) was determined after applying the peptide denoted "PS" in Figure 2. Figure 2A shows representative images of the inhibitory phosphorylated CRMP2 (pCRMP2$^{T514}$) of DRG (L6) from strep-treated mice after i.p. DMSO or peptide injection. The tissue was harvested 3 days after the injection. βIII-tubulin (Tub) served as a neuronal marker. Figure 2A shows a Western blot against pCRMP2$^{T514}$ of L4-L6 DRG from mice treated as described.

As can be taken from Figures 2A, 2B and 2C, TAT-fused P5 strongly reduced CRMP2 inactivation in DRG neurons. It is assumed that TAT-fused P5 can improve diabetic axonal regeneration.

To verify the regeneration-stimulating effect *in vivo,* axonal regeneration was determined. For this, intraperitoneal strep injection was induced in T1DM mice two months before sciatic nerve crush (SNC). The peptide of SEQ ID No. 13 (denoted P5) was intrathecally injected 1 day prior and on the day of the SNC. Anatomical regeneration was assessed 3 days post-injury (dpi).

Figure 2D shows representative images of axonal regeneration in a vehicle or strep-injected mice treated with DMSO or the peptide of SEQ ID No. 13 (denoted P5) either 1 day before or on the day of the sciatic nerve crush (SNC). Nerves were isolated 3 dpi and stained against choline acetyltransferase (ChAT) as a marker for motor fibers. Asterisks mark the crush site. Figure 2E shows the quantification of axon regeneration depicted in Figure 2D.

Figure 2F shows representative images of axon regeneration of sensory fibers stained against superior cervical ganglion 10 (SCG10) of the experimental conditions as described. Asterisks marking the crush site. Figure 2G shows the quantification of axon regeneration depicted in Figure 2F.

Figure 2H shows representative images of axon regeneration of sympathetic fibers stained with tyrosine hydroxylase (TH) in the experimental conditions described. Asterisks marking the crush site. Figure 2I shows the quantification of axon regeneration depicted in Figure 2H.

As can be taken from Figure 2, intrathecal injection of TAT-fused P5 (SEQ ID No. 13) accelerated anatomical regeneration of motor, sensory, and sympathetic fibers in the diabetic sciatic nerve.

These results show that intrathecal injection of TAT-fused P5 (SEQ ID No. 13) strongly reduced CRMP2 inactivation in DRG neurons (Fig. 10 A-C). Moreover, this treatment accelerated the anatomic regeneration of motor, sensory and sympathetic fibers in the diabetic sciatic nerve. Thus, also treatment with TAT-fused P5 can enable treatment of diabetic nerve damage.

**Claims**

1. A peptide comprising the amino acid sequence LVESCKEAFWDRCLSVINLMSSKM according to SEQ ID No. 1 wherein the peptide is linked to a trans-activator of transcription (TAT) domain, or a pharmaceutically acceptable salt thereof.

2. The peptide according to claim 1, wherein the TAT domain is selected from the group comprising YARAARRAARR (SEQ ID No. 2), YGRKKRRQRRR (SEQ ID No. 9), GRKKRRQRRR (SEQ ID NO: 10), GRKKRRQRRRPQ (SEQ ID NO: 11) and RKKRRQRRR (SEQ ID NO: 12).

3. The peptide according to claim 1 or 2, wherein the peptide comprises or consists of the amino acid sequence LVESCKEAFWDRCLSVINLMSSKMYARAARRAARR according to SEQ ID No. 3.

4. The peptide according to any one of claims 1 to 3 for use as a medicament.

5. The peptide according to any one of claims 1 to 3, for use in the treatment of axonal damage in diabetes.

6. A peptide selected from the group comprising P5 (SEQ ID No. 4), P4 (SEQ ID No. 5), P3 (SEQ ID No. 6), P2 (SEQ ID No. 7) and P1 (SEQ ID No. 8), where the peptide is linked to a trans-activator of transcription (TAT) domain, for use in the treatment of axonal damage in diabetes.

7. An expression vector expressing a nucleic acid encoding the peptide according to any one of claims 1 to 3, or expressing a nucleic acid encoding a peptide selected from the group comprising P5 (SEQ ID No. 4), P4 (SEQ ID No. 5), P3 (SEQ ID No. 6), P2 (SEQ ID No. 7) and P1 (SEQ ID No. 8), where the peptide is linked to a trans-activator of transcription (TAT) domain, for use in the treatment of axonal damage in diabetes.

8. The peptide for use according to claim 5 or 6, or the expression vector for use according to claim 7, wherein the axonal damage is diabetic peripheral neuropathy.

9. The peptide for use according to claim 5 or 6, or the expression vector for use according to claim 7, wherein the peptide or the vector is for use in promoting axonal regeneration in diabetic peripheral neuropathy.

10. A pharmaceutical composition comprising as an active ingredient a peptide according to any one of claims 1 to 3.

11. The pharmaceutical composition according to claim 10, or a pharmaceutical composition comprising a peptide selected from the group comprising P5 (SEQ ID No. 4), P4 (SEQ ID No. 5), P3 (SEQ ID No. 6), P2 (SEQ ID No. 7) and P1 (SEQ ID No. 8), where the peptide is linked to a trans-activator of transcription (TAT) domain, or a pharmaceutical composition comprising an expression vector expressing a nucleic acid encoding the peptide according to any one of claims 1 to 3, or expressing a nucleic acid encoding a peptide selected from the group comprising P5 (SEQ ID No. 4), P4 (SEQ ID No. 5), P3 (SEQ ID No. 6), P2 (SEQ ID No. 7) and P1 (SEQ ID No. 8), where the peptide is linked to a trans-activator of transcription (TAT) domain, for use in the treatment of axonal damage in diabetes.

12. The pharmaceutical composition according to claim 10 or 11, wherein the pharmaceutical composition is formulated for topical or local application such as intraneural or periradicular application, or systemic application such as intramuscular, intraperitoneal, intravenous, subcutaneous, intrathecal or oral application.

13. The pharmaceutical composition according to any one of claims 10 to 12, wherein the pharmaceutical composition is formulated for intraocular or topical application, such as in the form of eye drops.

14. Use of a peptide according to any one of claims 1 to 3, or use of a peptide selected from the group comprising P5 (SEQ ID No. 4), P4 (SEQ ID No. 5), P3 (SEQ ID No. 6), P2 (SEQ ID No. 7) and P1 (SEQ ID No. 8), where the peptide is linked to a trans-activator of transcription (TAT) domain, or use of an expression vector expressing a nucleic acid encoding the peptide according to any one of claims 1 to 3, or expressing a nucleic acid encoding a peptide selected from the group comprising P5 (SEQ ID No. 4), P4 (SEQ ID No. 5), P3 (SEQ ID No. 6), P2 (SEQ ID No. 7) and P1 (SEQ ID No. 8), where the peptide is linked to a trans-activator of transcription (TAT) domain, for the manufacture of a medicament for the treatment of axonal damage in diabetes.

15. A method of treating axonal damage in diabetes, the method comprising the step of administering to a subject a therapeutically effective amount of

a) a peptide according to any one of claims 1 to 3, or

b) a peptide selected from the group comprising P5 (SEQ ID No. 4), P4 (SEQ ID No. 5), P3 (SEQ ID No. 6), P2 (SEQ ID No. 7) and P1 (SEQ ID No. 8), where the peptide is linked to a trans-activator of transcription (TAT) domain, or

c) an expression vector expressing a nucleic acid encoding the peptide according to any one of claims 1 to 3, or expressing a nucleic acid encoding a peptide selected from the group comprising P5 (SEQ ID No. 4), P4 (SEQ ID No. 5), P3 (SEQ ID No. 6), P2 (SEQ ID No. 7) and P1 (SEQ ID No. 8), where the peptide is linked to a trans-activator of transcription (TAT) domain.

Figure 1

Figure 1

**H**

daily i.p. injection of 400 µg/kg AP-CDK5

-60 ⟋⟋ 0 1 4 7 9 14 21 28 35 dpi

strep SNC tissue isolation
* * * * * * * * * SSI/ von Frey

**I**

veh | strep
AP-CDK5 − | − +
pCRMP2^T514 — 70 kDa
β-actin — 55 kDa

**J**

■ veh ■ strep ▨ strep AP-CDK5

p=0.45
p=1·10⁻³ p=2·10⁻³

pCRMP2_T514 (norm.)

PBS AP-CDK5

**K**

dpi 0 1 7 14 35

veh PBS

strep PBS

AP-CDK5

Figure 1

Figure 2

Europäisches
Patentamt

European
Patent Office

Office européen
des brevets

# EUROPEAN SEARCH REPORT

Application Number

EP 23 18 6402

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/115790 A1 (PANT HARISH C [US] ET AL) 10 May 2012 (2012-05-10) | 14 | INV.<br>A61K38/16 |
| Y | * paragraphs [0031], [0033], [0040], [0117], [0142], [0186]; claims 1,11,15,17; sequences 1-5 * | 1-15 | C07K14/47<br>C07K14/16<br>A61P25/00<br>A61P25/02 |
| X | BK BINUKUMAR ET AL: "TFP5, a Peptide Derived from p35, a Cdk5 Neuronal Activator, Rescues Cortical Neurons from Glucose Toxicity",<br>JOURNAL OF ALZHEIMER`S DISEASE,<br>vol. 39, no. 4,<br>14 February 2014 (2014-02-14), pages 899-909, XP93100854,<br>NL<br>ISSN: 1387-2877, DOI: 10.3233/JAD-131784<br>Retrieved from the Internet:<br>URL:https://www.ncbi.nlm.nih.gov/pmc/articles/PMC4578662/pdf/nihms722259.pdf> | 14 | |
| Y | * the whole document * | 1-15 | |
| Y | KESAVAPANY SASHI ET AL: "Neuronal cyclin-dependent kinase 5: role in nervous system function and its specific inhibition by the Cdk5 inhibitory peptide",<br>BIOCHIMICA ET BIOPHYSICA ACTA (BBA) – PROTEINS & PROTEOMICS,<br>vol. 1697, no. 1, 2004, pages 143-153, XP085645286,<br>ISSN: 1570-9639, DOI: 10.1016/J.BBAPAP.2003.11.020<br>* page 150 * | 1-15 | TECHNICAL FIELDS SEARCHED (IPC)<br><br>A61K<br>C07K<br>A61P |

The present search report has been drawn up for all claims

| Place of search | Date of completion of the search | Examiner |
|---|---|---|
| Munich | 13 November 2023 | Weisser, Dagmar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 23 18 6402

This annex lists the patent family members relating to the patent documents cited in the above-mentioned European search report.
The members are as contained in the European Patent Office EDP file on
The European Patent Office is in no way liable for these particulars which are merely given for the purpose of information.

13-11-2023

| Patent document cited in search report | Publication date | Patent family member(s) | Publication date |
|---|---|---|---|
| US 2012115790 A1 | 10-05-2012 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8597660 B2

**Non-patent literature cited in the description**

- **ZHENG et al.** *Journal of Biological Chemistry*, 2010, vol. 285 (44), 34102-34212
- **BK BINUKUMAR et al.** *J Alzheimers Dis*, 2014, vol. 39 (4), 899-909
- **COLEMAN ; HUMMEL**. *Diabetologia*, 1967, vol. 3, 238-248
- **LEVIN, E et al.** *Sci Rep*, 2016, vol. 6, 1-10
- **GOBRECHT et al.** *Nature Communications*, 2014, vol. 5, 1-10
- **BAPTISTA AF et al.** *J Neurosci Methods*, 2007, vol. 161, 259-264